# EUROPEAN PATENT APPLICATION

(11) **EP 3 653 130 A2**
(43) Date of publication of application: **20.05.2020**
(21) Application number: 18832724.1
(22) Date of filing: 03.07.2018
(51) Int. Cl.: A61B 8/08

(54) **METHOD FOR DETECTING SPINAL DEFORMITY USING THREE-DIMENSIONAL ULTRASONIC IMAGING**

(30) Priority: 11.07.2017 CN 201710563513
(71) Applicant: Telefield Medical Imaging Limited, Hong Kong (CN)
(72) Inventor: ZHENG, Yongping, Hong Kong (HK)
(74) Representative: Hellmich, Wolfgang
(86) International application number: PCT/CN2018/094308
(87) International publication number: WO 2019/011158

(57) **Abstract**

The present application relates to a method for detecting spinal deformity using three-dimensional ultrasound imaging. A method for detecting spinal deformity using three-dimensional ultrasound imaging, wherein, comprising the following steps: S1. obtaining a three-dimensional image of a spine by a three-dimensional ultrasound imaging system; S2. obtaining axial rotation information of the spine through the three-dimensional image of the spine; S3. using the axial rotation information of the spine to adjust the three-dimensional image of the spine; S4. projecting the adjusted three-dimensional image of the spine after image on a coronal and / or sagittal plane to obtain a projection of the coronal and / or sagittal plane; S5. calculating the spinal deformity data by the projection of the coronal or sagittal plane. In the invention, the three-dimensional image is obtained through three-dimensional ultrasound imaging system; the rotation of vertebral body of three-dimensional image is obtained by analyzing rotation information and image information of each two-dimensional ultrasound image; and the rotation center of each two-dimensional image is estimated through spine statistical information. This method can more accurately measure the deformity angle of spine in each plane.

## Description

### TECHNICAL FIELD

The present application relates to a method for detecting spinal deformity using three-dimensional ultrasound imaging.

### BACKGROUND

In recent years, three-dimensional ultrasound imaging technology has been widely used in the measurement of human spinal deformity, such as scoliosis evaluation, and achieved good results. However, ultrasound can only obtain images at the posterior portion of the spine (i.e. from the back), so the images obtained mainly contain information about the transverse process, spinous process, and other spine bones at the posterior side. Because of the anatomical structure of the spine bone, when the main body of the spine bone has rotation, the structures obtained from the ultrasound image about the posterior portion of the spine bone will have a relatively large rotation distance, which greatly affects the measurement of spinal curvature. At the same time, in sagittal plane, the angle of anterio-posterioral curvature of spine will also be affected. This is also a deficiency between the measurement of spine curvature by ultrasound imaging and X-ray image detection. On the X-ray image, the information of the main body of the spine is detected, so the influence of rotation is relatively smaller, although the X-ray image itself cannot provide the measurement of rotation.

### SUMMARY

In order to solve the above problems, the application discloses a new method for detecting spinal deformity using three-dimensional ultrasound imaging, which uses the information about the rotation of spine obtained in three-dimensional ultrasound scanning to adjust the three-dimensional ultrasound image of spine, thus greatly reducing the error of deformity measurement in the coronal plane and sagittal plane, and can effectively calculate the rotation angle of spine.

To achieve the above purpose, the technical scheme adopted by the application is as follows:
A method for detecting spinal deformity using three-dimensional ultrasound imaging, wherein, comprising the following steps:
S1. obtaining a three-dimensional image of a spine by a three-dimensional ultrasound imaging system;
S2. obtaining axial rotation information of the spine through the three-dimensional image of the spine;
S3. using the axial rotation information of the spine to adjust the three-dimensional image of the spine;
S4. projecting the adjusted three-dimensional image of the spine after projecting on a coronal and / or sagittal plane to obtain a projection of the coronal and / or sagittal plane;
S5. calculating the spinal deformity data by the projection of the coronal or sagittal plane.

As preferred, the axial rotation information of the spine is obtained from rotation data of each two-dimensional ultrasound image forming the three-dimensional image of the spine in the axial direction of the spine.

As preferred, the two-dimensional ultrasound image is obtained by scanning skin on the back of a human body vertically by an ultrasound probe.

As preferred, the axial rotation information of the spine is obtained from three-dimensional space information of symmetrical characteristic areas on the left and right sides of each spine bone in the two-dimensional ultrasound image that constitutes the three-dimensional image of the spine, including left and right transverse processes, left and right articular processes, left and right vertebral arches, left and right vertebral lamina.

As preferred, image adjustment refers to correcting the axial rotation information of each spine bone with a selected rotation axis in the axial direction of the spine according to the axial rotation information of the spine at a specific angle, rotating every two-dimensional ultrasound image at a specific angle with a selected rotation axis in the axial direction of the spine according to the axial rotation information of the spine to correct the axial rotation of every corresponding vertebral bone, which making the rotation of each vertebral bone in the axial direction relative to a reference position zero.

As preferred, the selected rotation axis refers to a rotation axis of the spine in axial rotation.

As preferred, the selected rotation axis refers to an axial centerline of a vertebral body of the spine.

As preferred, a distance from the selected rotation axis to a body surface in the two-dimensional ultrasound image is obtained by analyzing a spine X-ray image, a CT image, or a magnetic resonance image of a subject in the same period.

As preferred, a transverse position of the selected rotation axis in the two-dimensional ultrasound image is determined by a position of an ultrasonic reflection signal of a spinous process or a position of an ultrasonic shadow area formed by the spinous process.

As preferred, a rotation amount of the specific angle is calculated by a relative distance between the position of an ultrasonic reflection of a spinous process and a position of the ultrasonic reflection of a vertebral body surface in the ultrasonic image and the distance between their projections on the coronal plane, and the ultrasonic reflection of the vertebral body surface is formed by an ultrasonic wave propagating to a surface of a vertebral body through a hole in the back of spine.

As preferred, the selected rotation axis is obtained by a preset formula about an age of the subject, a total length of the spine, and / or the size of each vertebral body, and a distance between the spinous process and the rotation axis.

As preferred, the size of the spine refers to a distance between left and right symmetrical feature points of the spine, or between the spinous process and other spine feature points or feature planes.

As preferred, the preset formula is obtained by counting the spine of a large number of people to obtain the size of each vertebral bone, the percentage of each vertebral bone in the total length of the spine, the correlation between the distance among feature points and height as well as age.

As preferred, following steps are also included between steps S3 and S4.

S3-1. marking a position of the selected rotation axis in a corresponding two-dimensional ultrasound image, i.e. marking with points, circles, lines, and / or other distinctive marks.

As preferred, after step S5, comprising the following steps:
S6. connecting positions of the selected rotation axis in all two-dimensional ultrasound images to form a three-dimensional curve, which contains deformity information of the spine on the coronal and sagittal planes.

As preferred, after step S5, comprising the following steps:
S7. connecting positions of the selected rotation axis in all two-dimensional ultrasound images to form a three-dimensional curve and using a series of lines perpendicular to the three-dimensional curve to represent the axial rotation of the spine.

As preferred, after step S5, comprising the following steps:
S8. determining whether the axial rotation of the spine has reached preset correction requirements, and if not, repeat steps S2 to S5.

As preferred, the correction requirements refer to that the projection of the position of the ultrasonic reflection of the spinous process in the three-dimensional ultrasound image and the projection of the position of ultrasound reflection on the surface of vertebral body on the coronal plane should be along the same line, and the ultrasonic reflection of the vertebral body surface is formed by an ultrasonic wave propagating to a surface of a vertebral body through a hole in the back of spine.

As preferred, between the steps S1 and S2, further comprising the following step:
S1-1. obtaining an axial rotation reference surface, which is a part of the human body that is relatively not easy to rotate and deform.

The beneficial effects of using the application are:
in the application the three-dimensional image is obtained through the three-dimensional ultrasound imaging system; the positions of the vertebral main body, the transverse process, and the spinous process are obtained through the three-dimensional image data; the three-dimensional image of the spine is adjusted by using the axial rotation information of the spine; after the adjustment, the projection of the predetermined plane can be made, and the rotation angle of the spine can be determined and calculated by the projection along the predetermined plane. This method can more accurately measure the deformity angle of spine in each plane.

### BRIEF DESCRIPTION OF THE DRAWINGS

Figure 1 is a procedure block diagram of a method for detecting spinal deformation by three-dimensional ultrasound imaging of the application.
Figure 2 is a schematic of the spine.
Figure 3 is a two-dimensional ultrasound image of the spine.
Figure 4 shows the scanning profile of three-dimensional ultrasound imaging in the sagittal direction.
Figure 5 shows the scanning outline of the three-dimensional ultrasound image viewed axially from the bottom of spine, and the rotation of the ultrasound image in different positions can be clearly seen.
Figure 6a is an ultrasound image of the spine obtained by the ultrasound imaging device of the present application.
Figure 6b is a projection diagram of the coronal plane of a three-dimensional ultrasound imaging of the application.
Figure 6c is a projection diagram of the sagittal plane of the three-dimensional ultrasound imaging of the present application.
Figure 7 is a continuous point graph after the image features of the spinous process and transverse process obtained by the multi section spines in the application are obtained.
Figure 8 is an effect diagram of the continuous point-shaped image obtained after the image characteristics of the spinous process and the transverse process are attached to the three-dimensional simulation model of the spine.
Figure 9 is a schematic diagram of a calculation method for the rotation angle of the spine in the application.
Figure 10 is an effect diagram of projecting the three-dimensional image features of the vertebral main body and the three-dimensional image features of the spinous process and the transverse process to the same plane.
Figure 11 shows the location of the spine hole.
Figure 12 is the projection diagram of the spinous process and the holes between the two adjacent vertebrae in the vertical state.
Figure 13 shows the projection diagram of the spinous process and the holes between the two adjacent vertebrae in the bending state.
Figure 14 shows the projection diagram of the spinous process and the holes between the two adjacent vertebrae when the spine is rotated and bent.
Figure 15 is a three-dimensional curve consisting of the rotation axis of each position, plus the rotation amount of each position represented by a straight line.

### DETAILED DESCRIPTION OF THE PREFERRED EMBODIMENT

The application will be described in detail below in combination with the accompanying drawings.

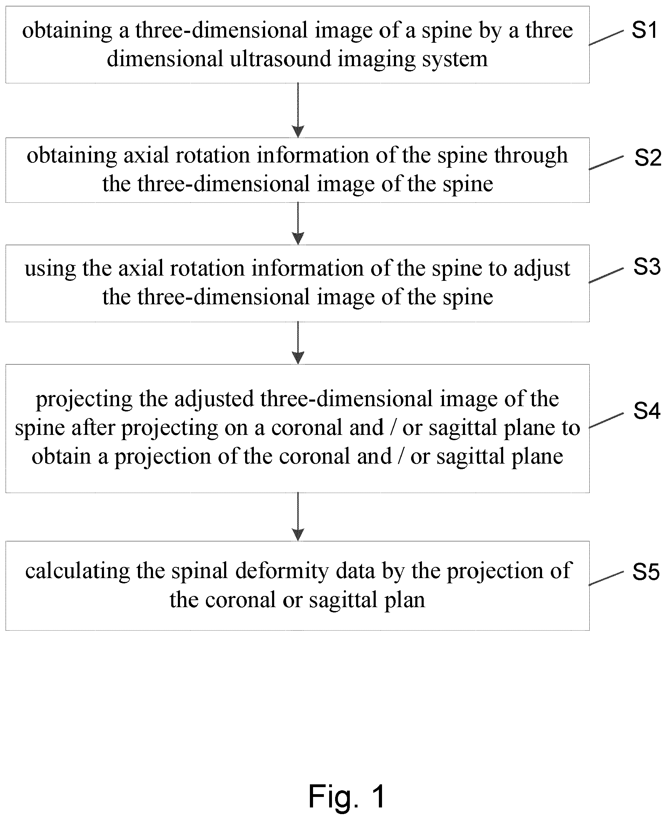

As shown in Figure 1, the present application provides a method for detecting spinal deformity using three-dimensional ultrasound imaging, wherein, comprising the following steps: S1. obtaining a three-dimensional image of a spine by a three-dimensional ultrasound imaging system; S2. obtaining axial rotation information of the spine through the three-dimensional image of the spine; S3. using the axial rotation information of the spine to adjust the three-dimensional image of the spine; S4. projecting the adjusted three-dimensional image of the spine after image on a coronal and / or sagittal plane to obtain a projection of the coronal and / or sagittal plane; S5. calculating the spinal deformity data by the projection of the coronal or sagittal plane.

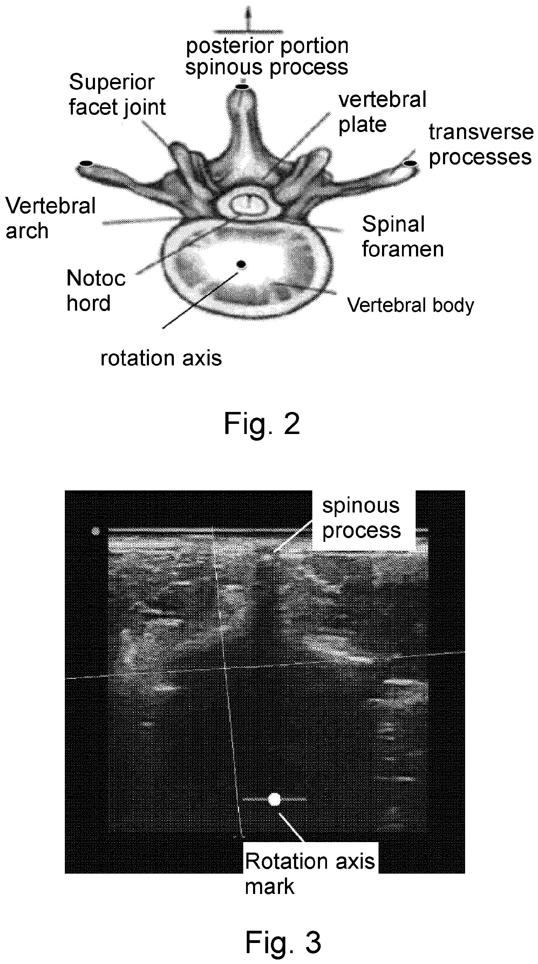

Figure 2 is a cross-sectional view of the vertebral body, showing the location of the spinous process and the transverse process and the axis where the rotation axis is located. In this embodiment, the position of the axis where the rotation axis is located is the center of the vertebral body. Fig. 3 is a two-dimensional image of the vertebrae. In this image, it can be seen that the position of the spinous process is located at the top tip of the black shadow part, and the vertebral body is a quasi circular area in the black shadow part. The rotation axis is at the corresponding position of the vertebral body. In the two-dimensional ultrasound image of Fig. 3, the horizontal line is a reference surface without rotation, that is, the two-dimensional ultrasound image shown has been rotated.

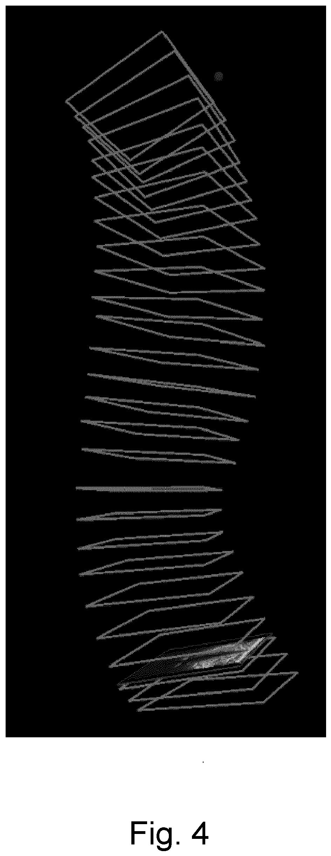

The three-dimensional ultrasound image in the sagittal direction of the scanning contour is shown in Figure 4. In the three-dimensional ultrasound image, the three-dimensional scanning image in the direction of the sagittal plane consists of several two-dimensional scanning images arranged in a continuous order. The two-dimensional image is arranged in the direction of the natural curvature of the spine, and the two-dimensional image in the image is perpendicular to the natural curvature of the spine.

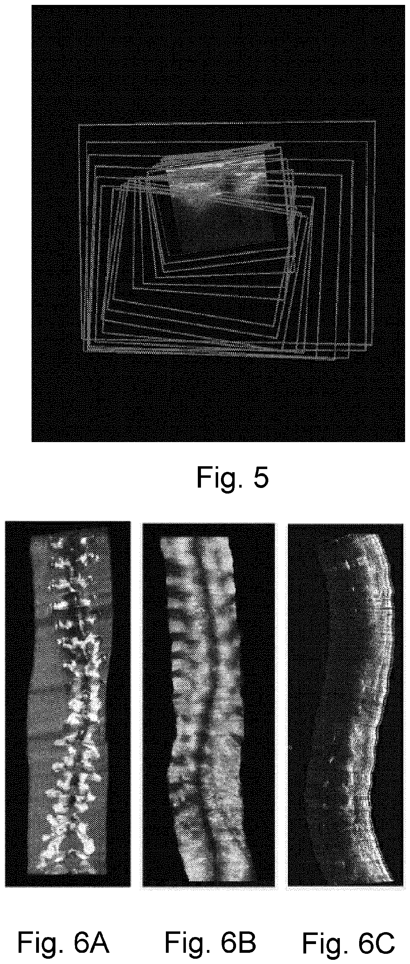

As shown in Fig. 5, the rotation of ultrasonic image in different positions can be clearly seen in the scanning profile of three-dimensional ultrasound imaging viewed axially from the bottom to the top. Image adjustment refers to correcting the axial rotation information of each vertebral bone with a selected rotation axis in the axial direction of the spine according to the axial rotation information of the spine at a specific angle, rotating each two-dimensional ultrasound image at a specific angle with a selected rotation axis in the axial direction of the spine according to the axial rotation information of the spine to correct the axial rotation of each vertebral bone, which making the rotation of each spine in the axial direction relative to a reference position zero. One of the purposes of the correction is to rotate the image at the position with rotation reversely, that is, achieving all images looking like not rotated.

The selected rotation axis can have multiple reference axes, and only one rotation axis can be selected in each measurement. In one embodiment, the selected rotation axis refers to a rotation axis of the spine in axial rotation. In one embodiment, the selected rotation axis refers to an axial centerline of vertebral bodies of the spine.

Understandably, a distance from the selected rotation axis to a body surface in the two-dimensional ultrasound image is obtained by analyzing a spine X-ray imaging, a CT image, or a magnetic resonance image of a subject collected in the same period. The selected rotation axis has synchronicity to ensure the accuracy of measurement data, and corresponds to the acquired two-dimensional ultrasound image.

In this embodiment, a transverse position of the selected rotation axis in the two-dimensional ultrasound image is determined by a position of an ultrasonic reflection signal of a spinous process or a position of an ultrasonic shadow area formed by the spinous process.

The axial rotation information of the spine is obtained from rotation data of each two-dimensional ultrasound image forming the three-dimensional image of the spine in the axial direction of the spine. In this embodiment, the rotation information of each two-dimensional image, i.e. the rotation information of the spine, can be determined by the rotation data of the two-dimensional image in the axial direction of the spine in Figure 4 and Figure 5. The two-dimensional ultrasound image is obtained by scanning on the back of a human body vertically by an ultrasound probe which is closely contacted with the skin, in order to minimize the error of obtaining the two-dimensional ultrasound image, and to ensure the clarity of the two-dimensional ultrasound image, and to ensure the axial rotation of the ultrasound image in the axial direction is the axial rotation of the spine.

The axial rotation information of the spine is obtained from three-dimensional spatial information of areas with symmetrical features on the left and right sides of each vertebral bone in the two-dimensional ultrasound image that constitutes the three-dimensional image of the spine, including left and right transverse processes, left and right articular processes, left and right vertebral arches, left and right vertebral lamina. In the two-dimensional ultrasound image, reference Figure 2 is the cross-sectional structure of the spine corresponding to the two-dimensional ultrasound image to obtain the axial rotation information of the spine.

Specifically, as shown in Figure 6A, in the method for detecting spinal deformity using three-dimensional ultrasound imaging provided in this embodiment, first, obtaining the three-dimensional image through the three-dimensional ultrasound imaging system. As shown in Figure 6A, if the gray level in the image is different, the three-dimensional image features can be acquired automatically by software, that is, the vertical strip area with large gray level change in the middle is the area of the spine, the black origin in the middle of the image is the image position of the spinous process of the spine, and the white wing end position on both sides of the image position of the spinous process is the position of the transverse process. Fig. 6C is a continuous point-shaped image after acquiring the image features of the spine on the sagittal plane, that is, a schematic diagram of the image features obtained by the method for detecting spinal deformity using three-dimensional ultrasound imaging.

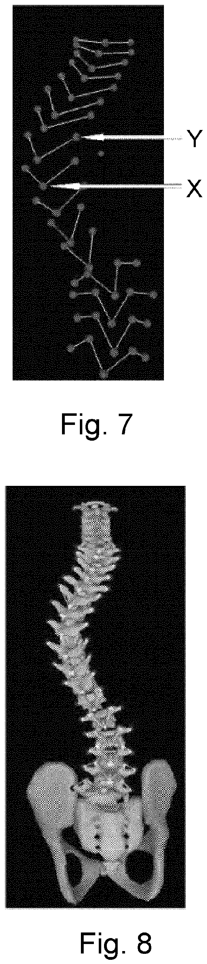

As shown in Fig. 6B, the three-dimensional image features are then projected onto the coronal and / or sagittal planes. In this embodiment, the projection of a two-dimensional image through a coronal plane is described in detail. Three-dimensional image features are projected onto the coronal plane to form image features drawn by points and lines, as shown in Figure 7.

Finally, the data of the spinal deformity is calculated by the position difference of the three-dimensional image features in the coronal and / or sagittal plane and the projection plane. The calculation method of the data of the spinal deformity in this embodiment is described in detail below.

As preferred, the characteristics of the three-dimensional image of spine are imported into the pre saved database, in which the three-dimensional image model is formed and displayed by the characteristics of the three-dimensional image of spine. As shown in Figure 7, the three-dimensional image characteristics of the acquired spine are shown as the original point shape. In Figure 7, the origin of X direction is the spinous process, and the origin of Y direction is the transverse process. The model of the three-dimensional image of the spine in this embodiment is shown in Fig. 8. Part X of the three-dimensional image characteristics of the spinous process in Fig. 7 is attached to the position of the spinous process of the model, and part Y of the three-dimensional image characteristics of the transverse process in Fig. 7 is attached to the position of the transverse process of the model. The three-dimensional model forms a three-dimensional simulation diagram that can be observed intuitively. This three-dimensional simulation can enlarge and reduce the image, and rotate the viewing angle, so that patients or doctors can observe the state of spine easily. The spine model stored in the database in this embodiment can be divided into multiple types according to the patient's gender, height, age, etc. before the spine model is retrieved, the actual gender, height, age and other parameters of the patient can be pre inputted to call out the corresponding spine model.

The rotation axis is obtained by a preset formula about an age of the subject, a total length of the spine, and / or the size of each vertebral bone and a distance between the spinous process and the rotation axis. The size of the spine refers to a distance between left and right symmetrical feature points of the spine, or between the spinous process and other spine feature points or feature planes. The preset formula is obtained by counting the spine of a large number of people to obtain the representing size of each vertebral bone, a percentage of each vertebral bone in the total length of the spine, the correlation between a distance among features points and the height as well as age.

The determination scheme of the rotation axis, i.e. the preset relationship, can match the position of the rotation axis with the different physiological characteristics of the examinee as much as possible.

As preferred, the three-dimensional image characteristics of the spine include the three-dimensional image features of the vertebral body of the spine and the spinous process / transverse process. Three-dimensional image features of spine include three-dimensional spatial position data and angle data. As shown in Figure 7, after obtaining the three-dimensional image features of the vertebral body of the spine and the spinous process, the angle data of the relative position relationship between the spinous process and the transverse process is calculated through the image features.

In this embodiment, the reflected signal of the vertebral body surface of the spine can also be fused with the ultrasonic reflected signal of other parts, such as the spinous process, the transverse process and other parts, to measure the deformity and rotation of the spine in various planes.

If the spine is not rotated, the two will overlap in the same shape, but at different depths. In the case that the spine is rotated, the curved surface composed of the reflected signals of each spinous process has different angles with the curve composed of the reflected signals of the vertebral body surface of the spine bone. The angle of the curve formed by the spinous process is reduced because the displacement of the spinous process caused by rotation counteracts the movement caused by some portion of side bending.

A rotation size of the specific angle is calculated by a relative distance between a position of an ultrasonic reflection of a spinous process and a position of an ultrasonic reflection of a vertebral surface in the ultrasonic image and the distance between their projections on the coronal plane, and the ultrasonic reflection of the vertebral body surface is formed by an ultrasonic wave propagating to a surface of a vertebral body through a hole in the back of spine.

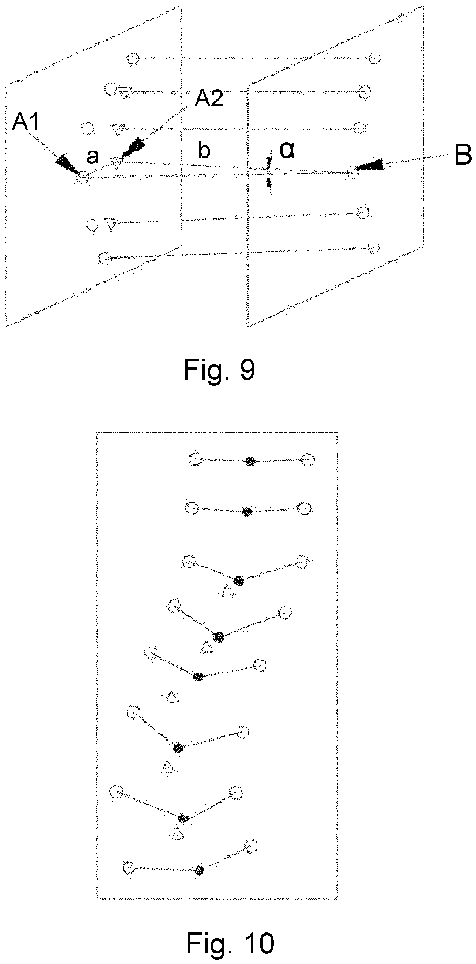

Project the three-dimensional image features of the main body of the spine and the three-dimensional image features of the spinous process / transverse process into the same plane. If the line of the projection of the main body of the spine is not overlapped with the line of the projection of the spinous process / transverse process, the spine is judged to have rotation. As shown in Figure 9, the hollow circle is the transverse process; the solid circle is the spinous process; the hollow triangle is the opposite angle of the main surface of the spine. If the curve formed by the spinous process in the shape of solid circle does not overlap with the curve formed by the surface reflection of the main body of the spine bone in the shape of hollow triangular, it means that the spine bone has been rotated, and the degree of rotation of each spine bone can be calculated according to the above method. That is to say, the three-dimensional image features of the spinous process are projected on one plane, and the reflection features of the main surface of the spine are projected on the other. The rotation degree of each section of the vertebral bone is calculated according to the specific two planes and the projection horizontal position relationship. The angle α is calculated by the length of a and b.

As shown in Figures 10-12, the three-dimensional image features of the spine also include the three-dimensional image features of the holes between the adjacent two spine bones. Project the three-dimensional image features of the spine body and the three-dimensional image features of the holes into the same plane. If the line of the projection of the spine body and the line of the projection of the holes do not overlap, it is determined that the spine has rotation.

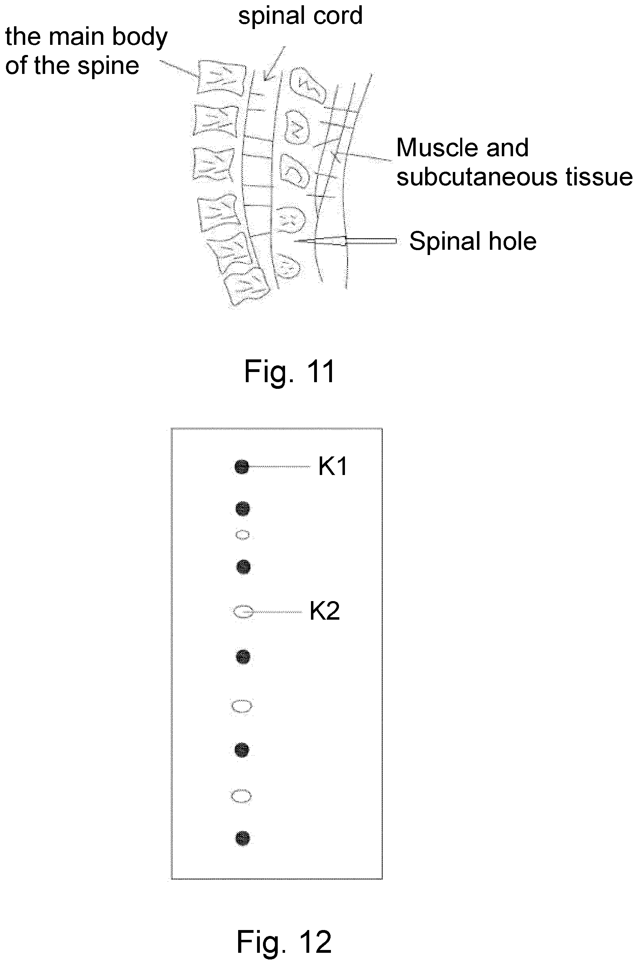

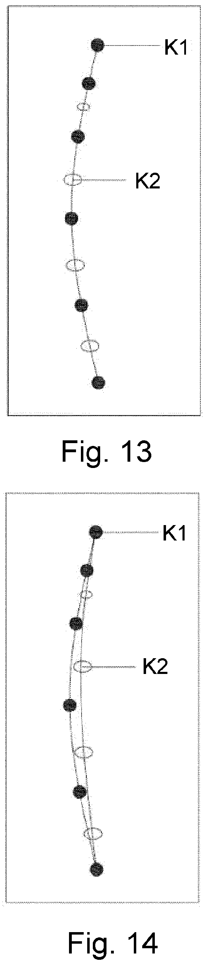

In Figure 11, the positions of the holes between the adjacent two vertebrae are shown. In general, the holes, the spinous process and the vertebral body of the spine are projected along the same straight line. Figure 12-14 shows the projection of the spine on the coronal plane and the holes between the two adjacent vertebrae, in which K1 represents the spinous process and K2 represents the holes between the two adjacent vertebrae. As shown in Figure 12, without rotation and bending of the spine, the spinous process and the hole are along the same line. As shown in Figure 13, when the spinous process and the hole are in the same arc, it means the spine is bent. As shown in Figure 14, when the line of the spinous process and the line of the hole are not on the same arc, it means that the spine is bent and rotated.

According to the depth of the hole between the spine and the surface of the body, the three-dimensional image features of the hole were modified. In addition, the method of calculating the rotation angle can also make some modifications, that is, to calculate the depth of the cavity, rather than the vertebral body surface of the spine, because the movement of the reflection area of the vertebral body surface of the spine actually comes from the movement of the hole.

Between steps S3 and S4, following steps are also included: S3-1. marking a position of the selected rotation axis in a corresponding two-dimensional ultrasound image, i.e. marking with points, circles, lines, and / or other distinctive marks.

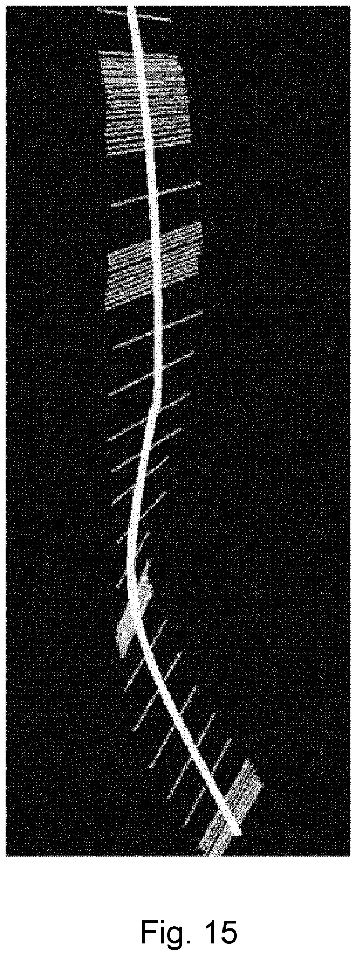

After step S5, comprising the following steps S6. connecting positions of the selected rotation axis in all two-dimensional ultrasound images to form a three-dimensional curve, which contains deformity information of the spine on the coronal and sagittal planes, for example, obtaining the line simulating the central position of the spinous process, the transverse process and vertebral body of the spine. After step S5, comprising the following steps S7. connecting positions of the selected rotation axis in all two-dimensional ultrasound images to form a three-dimensional curve and using a series of lines perpendicular to the three-dimensional curve to represent the axial rotation of the spine. The axial rotation of the spine is determined, as shown in Figure 15, corresponding to the lateral position relationship and distance of the above lines.

After step S5, comprising the following steps: S8. determining whether the axial rotation of the spine has reached preset correction requirements, and if not, repeat steps S2 to S5, ensuring the accuracy of the measurement. If the correction requirements are not met, the ultrasonic image needs to be obtained repeatedly. The correction requirements refer to the minimization of the distance, i.e. along the same line, between the projection of the position of the ultrasonic reflection of the spinous process in the three-dimensional ultrasound image and the projection of the position of ultrasound reflection on the surface of vertebral body on the coronal plane, and the ultrasonic reflection of the vertebral surface is formed by an ultrasonic wave propagating to a surface of a vertebral body through a hole in the back of spine.

Between the steps S1 and S2, further comprising the following step:S1-1. obtaining an axial rotation reference surface, which is a part of the human body that is relatively not easy to rotate and deform. For example, the bottom of the back and the part near the hip are not easy to rotate.

The above content is only a preferred embodiment of the application. For those skilled in the art, according to the idea of the application, many changes can be made in the specific implementation mode and application scope. As long as these changes are not divorced from the concept of the application, they belong to the protection scope of the application.

## Claims

1. A method for detecting spinal deformity using three-dimensional ultrasound imaging, wherein, comprising following steps:
S1. obtaining a three-dimensional image of a spine by a three-dimensional ultrasound imaging system;
S2. obtaining axial rotation information of the spine through the three-dimensional image of the spine;
S3. using the axial rotation information of the spine to adjust the three-dimensional image of the spine;
S4. projecting the adjusted three-dimensional image of the spine after projecting on a coronal and / or sagittal plane to obtain a projection of the coronal and / or sagittal plane;
S5. calculating spinal deformity data by the projection of the coronal or sagittal plane.

2. The method for detecting spinal deformity using three-dimensional ultrasound imaging according to claim 1, wherein: the axial rotation information of the spine is obtained from rotation data of each two dimensional ultrasound image forming the three-dimensional image of the spine in an axial direction of the spine.

3. The method for detecting spinal deformity using three-dimensional ultrasound imaging according to claim 2, wherein: the two dimensional ultrasound image is obtained by scanning a back of a human body vertically by an ultrasound probe contacting on skin.

4. The method for detecting spinal deformity using three-dimensional ultrasound imaging according to claim 1, wherein: the axial rotation information of the spine is obtained from three-dimensional spatial information of areas with symmetrical features on left and right sides of each vertebral bone in the two-dimensional ultrasound image that constitutes the three-dimensional image of the spine, including left and right transverse processes, left and right articular processes, left and right vertebral arches, left and right vertebral lamina.

5. The method for detecting spinal deformity using three-dimensional ultrasound imaging according to claim 1, wherein: image adjustment refers to correcting an axial rotation information of each vertebral bone with a selected rotation axis in the axial direction of the spine according to the axial rotation information of the spine at a specific angle, rotating each two-dimensional ultrasound image at a specific angle with a selected rotation axis in the axial direction of the spine according to the axial rotation information of the spine to correct the axial rotation of each vertebral bone, which making the rotation of each vertebral bone be zero in the axial direction relative to a reference position.

6. The method for detecting spinal deformity using three-dimensional ultrasound imaging according to claim 5, wherein: the selected rotation axis refers to a rotation axis of the spine in axial rotation.

7. The method for detecting spinal deformity using three-dimensional ultrasound imaging according to claim 5, wherein: the selected rotation axis refers to an axial centerline of a vertebral body of the spine.

8. The method for detecting spinal deformity using three-dimensional ultrasound imaging according to claim 5, wherein: a distance from the selected rotation axis to a body surface in the two-dimensional ultrasound image is obtained by analyzing a spine X-ray image, a CT image or a magnetic resonance image of a subject in the same period.

9. The method for detecting spinal deformity using three-dimensional ultrasound imaging according to claim 5, wherein: a transverse position of the selected rotation axis in the two-dimensional ultrasound image is determined by a position of an ultrasonic reflection signal of a spinous process or a position of an ultrasonic shadow area formed by the spinous process.

10. The method for detecting spinal deformity using three-dimensional ultrasound imaging according to claim 5, wherein: a rotation amount of the specific angle is calculated by a relative distance between a position of an ultrasonic reflection of a spinous process and a position of an ultrasonic reflection of a vertebral body surface in the ultrasonic image and the distance between their projections on the coronal plane, and the ultrasonic reflection of the vertebral body surface is formed by an ultrasonic wave propagating to a surface of a vertebral body through a hole in a back of spine.

11. The method for detecting spinal deformity using three-dimensional ultrasound imaging according to claim 5, wherein: the selected rotation axis is obtained by a preset formula about an age of a subject, a total length of the spine, and / or a size of each spine and a distance between a spinous process and a rotation axis.

12. The method for detecting spinal deformity using three-dimensional ultrasound imaging according to claim 11, wherein: the size of the spine refers to a distance between left and right symmetrical feature points of the spine, or between the spinous process and other spine feature points or feature planes.

13. The method for detecting spinal deformity using three-dimensional ultrasound imaging according to claim 11, wherein: the preset formula is obtained by counting the spine of a large number of people to obtain the size of each vertebral bone, a percentage of each vertebral bone in the total length of the spine, a correlation between the distance among feature points and height as well as age.

14. The method for detecting spinal deformity using three-dimensional ultrasound imaging according to claim 1, wherein: following steps are also included between steps S3 and S4
S3-1. marking a position of a selected rotation axis in a corresponding two-dimensional ultrasound image, i.e. marking with points, circles, lines, and / or other distinctive marks.

15. The method for detecting spinal deformity using three-dimensional ultrasound imaging according to claim 1, wherein: after step S5, comprising following steps
S6. connecting positions of a selected rotation axis in all two-dimensional ultrasound images to form a three-dimensional curve, which contains deformity information of the spine on the coronal and sagittal planes.

16. The method for detecting spinal deformity using three-dimensional ultrasound imaging according to claim 1, wherein: after step S5, comprising following steps
S7. connecting positions of a selected rotation axis in all two-dimensional ultrasound images to form a three-dimensional curve and using a series of lines perpendicular to the three-dimensional curve to represent an axial rotation of the spine.

17. The method for detecting spinal deformity using three-dimensional ultrasound imaging according to claim 1, wherein: after step S5, comprising the following steps:
S8. determining whether the axial rotation of the spine has reached preset correction requirements, and if not, repeat steps S2 to S5.

18. The method for detecting spinal deformity using three-dimensional ultrasound imaging according to claim 17, wherein: the correction requirements refer to that the projection of a position of a ultrasonic reflection of a spinous process in the three-dimensional ultrasound image and the projection of the position of ultrasound reflection on the surface of vertebral body on the coronal plane should be calculated by a mutual distance calculation along the same line, and the ultrasonic reflection of the vertebral body surface is formed by an ultrasonic wave propagating to a surface of a vertebral body through a hole in a back of spine.

19. The method for detecting spinal deformity using three-dimensional ultrasound imaging according to claim 1, wherein: between steps S1 and S2, further comprising the following step:
S1-1. obtaining an axial rotation reference surface, which is a part of a human body that is relatively not easy to rotate and deform.
